(19) Europäisches Patentamt
European Patent Office
Office européen des brevets

(11) Veröffentlichungsnummer: **0 064 194**
**A2**

# (12) EUROPÄISCHE PATENTANMELDUNG

(21) Anmeldenummer: **82103209.1**

(22) Anmeldetag: **16.04.82**

(51) Int. Cl.³: **C 07 D 493/04**
//(C07D493/04, 307/00, 307/00)

(30) Priorität: **05.05.81 DE 3117612**

(43) Veröffentlichungstag der Anmeldung:
**10.11.82 Patentblatt 82/45**

(84) Benannte Vertragsstaaten:
**CH DE FR GB IT LI SE**

(71) Anmelder: **CASSELLA Aktiengesellschaft**
**Hanauer Landstrasse 526**
**D-6000 Frankfurt am Main 61(DE)**

(72) Erfinder: **Schönafinger, Karl, Dr.**
**Parkstrasse 1**
**D-8531 Uehfeld(DE)**

(74) Vertreter: **Urbach, Hans-Georg, Dr. et al,**
**Hanauer Landstrasse 526**
**D-6000 Frankfurt am Main 61(DE)**

## (54) Verfahren zur Herstellung von Isosorbid-5-nitrat.

(57) Bei dem Verfahren zur Herstellung von Isosorbid-5-nitrat wird Isosorbid mit einer aliphatischen Carbonsäure unter Bildung eines Acylierungsgemisches umgesetzt, das Acylierungsgemisch wird anschließend nitriert und das erhaltene Nitrierungsgemisch zur Abspaltung der Acylgruppen hydrolysiert und/oder umgeestert.

EP 0 064 194 A2

Croydon Printing Company Ltd.

Verfahren zur Herstellung von Isosorbid-5-nitrat

Die vorliegende Erfindung betrifft ein Verfahren zur Herstellung von 1,4-3,6-Dianhydro-D-sorbit-5-nitrat der Formel I

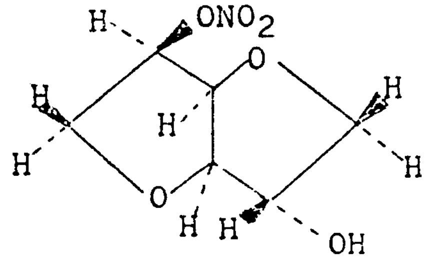

(I)

1,4-3,6-Dianhydro-D-sorbit-5-nitrat der Formel I wird auch als Isosorbid-5-nitrat oder als 5-ISM (Isosorbid-5-mononitrat) bezeichnet. Es wird als Heilmittel zur Behandlung von Herzkrankheiten, z.B. von Angina pectoris, eingesetzt. Bei der direkten Nitrierung von 1,4-3,6-Dianhydro-D-sorbit, auch Isosorbid genannt, nach I.G.Csizmadia und D.L.Hayward, Photochem. Photobiol. 4, 657 (1965) fällt ein Gemisch aus Nitraten an, in dem das Isosorbid-5-nitrat nur als untergeordneter Bestandteil anwesend ist. Bei der säulenchromatographischen Isolierung werden deshalb auch nur geringe Ausbeuten an Isosorbid-5-nitrat erhalten.

Bei einem anderen Herstellungsverfahren wird zunächst 1,4-3,6-Dianhydro-D-sorbit-2,5-dinitrat, auch Isosorbid-2,5-dinitrat genannt, hergestellt und dieses dann partiell verseift. Aus dem entstehenden Verseifungsgemisch, das Isosorbid-2,5-dinitrat, Isosorbid-5-nitrat, Isosorbid-2-nitrat und Isosorbid enthält, müssen die einzelnen Bestandteile, wie bei dem zuerst genannten Verfahren, durch aufwendige Verfahren voneinander getrennt werden (vgl. Anteunis et al, Org.Magnetic Resonance Vol. 3, 363 ff., (1971), D.L.Hayward et al, Can.J.Chem. 45, 2191 ff. (1967)).

Bei einem Verfahren zur Herstellung von 1,4-3,6-Dianhydro-D-glucitolmononitratestern (andere Bezeichnung für 1,4-3,6-Dianhydro-D-sorbitmononitratester) werden die als Ausgangsprodukte benötigten Isosorbid-2- und -5-nitrate durch direkte Nitrierung von Isosorbid hergestellt (vgl. DE-OS 2 751 934). Das Nitrierungsgemisch wird mit Eiswasser behandelt und anschließend mit Ether extrahiert. Der Etherextrakt wird unter Zugabe von Wasser konzentriert und die erhaltene wäßrige Lösung gefriergetrocknet. Durch Kolonnenchromatographie des Nitratgemisches erhält man das Isosorbid-5-nitrat in einer Ausbeute von rund 20 Gew.%.

Bei einem Verfahren zur Herstellung des zu dem Isosorbid-5-nitrat isomeren Isosorbid-2-nitrats wird Isosorbid mit einem Niedrigalkansäureanhydrid oder -chlorid oder -bromid acyliert (DE-OS 29 03 927), dann der Isosorbid aus dem Acylierungsgemisch extrahiert, um bei der nachfolgenden Nitrierung die Bildung des explosionsgefährlichen Isosorbid-2,5-dinitrats zu verhindern. In einer dritten Stufe wird das Acylierungsgemisch nitriert und das erhaltene Gemisch aus Isosorbid-5-acylat-2-nitrat, Isosorbid-2-acylat-5-nitrat und Isosorbid-2,5-diacylat mit einer anorganischen Base hydrolysiert. In dem Hydrolysierungsgemisch aus Isosorbid-2-nitrat, Isosorbid-5-nitrat und Isosorbid ist das Verhältnis Isosorbid-2-nitrat : Isosorbid-5-nitrat größer als 2 : 1, d.h. das Isosorbid-2-nitrat ist in mindestens doppelt so großer Menge vorhanden als das Isosorbid-5-nitrat. Aus dem Hydrolysierungsgemisch wird das Isosorbid-2-nitrat durch Kristallisation isoliert.

Schließlich ist noch ein Verfahren zur Herstellung von Isosorbid-5-nitrat bekannt (DE-OS 29 03 927), das von 1,4-3,6-Dianhydro-mannit, auch Isomannid genannt, ausgeht. Dabei wird Isomannid zunächst mit einem Säurehalogenid einer aromatischen, gegebenenfalls substituierten Benzol- oder Naphthalinsulfonsäure oder einem Säurehalogenid einer Perfluor-$C_{1-4}$-niederalkansulfonsäure, einer

$C_{1-4}$-Niederalkansulfonsäure oder einer Perfluor-$C_{1-4}$-niederalkancarbonsäure, einer Carbaminsäure oder der Schwefligsäure umgesetzt. Anstelle der Säurechloride können dabei im Fall der genannten Sulfonsäure, der Perfluor-$C_{1-4}$-niederalkansulfonsäure, der $C_{1-4}$-Niederalkansulfonsäure oder der Perfluor-$C_{1-4}$-niederalkancarbonsäure auch die entsprechenden Anhydride eingesetzt werden. Der erhaltene Isomannid-2-ester wird dann mit einem Alkali- oder Ammoniumsalz einer gegebenenfalls substituierten Benzoesäure oder einer $C_{1-4}$-Niederalkancarbonsäure oder der Ameisensäure umgesetzt, die 5-Hydroxygruppe des erhaltenen Isosorbid-2-esters mit Salpetersäure verestert und das erhaltene Isosorbid-2-ester-5-nitrat selektiv hydrolysiert und/oder umgeestert.

Die bisher bekannten Herstellungsverfahren für Isosorbid-5-nitrat sind vielstufig, be-inhalten umständliche und aufwendige Reinigungsoperationen oder liefern das gewünschte Produkt nur in unbefriedigender Ausbeute oder gehen zudem - wie im Falle des Iso-mannids - von nicht handelsüblichen Ausgangsprodukten aus.

Bei dem Verfahren der vorliegenden Erfindung wird Isosorbid-5-nitrat, ausgehend von Isosorbid, in einem relativ einfachen Verfahren und in relativ hoher Ausbeute hergestellt. Bei dem erfindungsgemäßen Verfahren zur Herstellung von 1,4-3,6-Dianhydro-D-sorbit-5-nitrat der Formel I, auch Isosorbid-5-nitrat genannt, wird 1,4-3,3-Dianhydro-D-sorbit, auch Isosorbid genannt, mit einer aliphatischen Carbonsäure umgesetzt, das erhaltene Acylierungsgemisch nitriert, anschließend die Acylgruppe durch Hydrolyse und/oder Umesterung abgespaltet und das Isosorbid-5-nitrat isoliert.

Normalerweise werden aliphatische Carbonsäuren mit 1 bis 7 C-Atomen für die Umsetzung mit dem Isosorbid verwandt. Derartige aliphatische Carbonsäuren sind z.B.: Ameisen-,

Essig-, Propion-, Butter-, Isobutter-, Valerian-, Isovalerian-, Capron- oder Önanthsäure, ferner Acryl-, Methacryl-, cis- oder trans-Croton-, Vinylessig- oder Angelicasäure oder ein Gemisch der genannten Säuren. Alkancarbonsäuren sind bevorzugt, insbesondere solche mit 2 bis 4 C-Atomen (Essig-, Propion-, Butter- oder Isobuttersäure). Vorzugsweise wird Essigsäure verwendet.

Die Umsetzung zwischen dem Isosorbid und der aliphatischen Carbonsäure wird ohne Anwesenheit eines Lösungsmittels, vorzugsweise aber in Anwesenheit eines geeigneten Lösungsmittels, durchgeführt. Bevorzugte Lösungsmittel sind solche, die ein ausreichendes Lösungsvermögen für Isosorbid besitzen und gleichzeitig die Abtrennung des bei der Veresterung des Isosorbids mit der aliphatischen Carbonsäure entstehenden Reaktionswassers durch azeotrope Destillation ermöglichen. Derartige bevorzugte Lösungsmittel sind z.B. Chloroform, Tetrachlorkohlenstoff, Ethylenchlorid, Chlorbenzol, Dimethoxyethan, Diethylenglycoldimethylether, Toluol. Auch Mischungen verschiedener Lösungsmittel können verwendet werden, insbesondere z.B. von Chlorbenzol und Toluol, oder von Ethylenglykoldimethylether und Toluol.

Die Umsetzung des Isosorbids mit der aliphatischen Carbonsäure wird in Gegenwart eines nicht oxidierenden, sauer reagierenden Katalysators durchgeführt. Derartige Katalysatoren sind z.B. Sulfonsäuren, wie z.B. p-Toluolsulfonsäure, Ethansulfonsäure, Methansulfonsäure; nichtoxidierende Mineralsäuren, wie z.B. Schwefelsäure, Salzsäure, Bromwasserstoffsäure, Phosphorsäure; sauer reagierende Salze, wie z.B. Alkalihydrogensulfate, wie z.B. Natriumhydrogensulfat. Anstelle der freien Säuren können auch mit Säuren beladene Ionenaustauscher bzw. Säuregruppen enthaltende polymere Verbindungen eingesetzt werden.

In der Regel kommen, bezogen auf Isosorbid, 0,1 bis 15 Gew.%, vorzugsweise 2 bis 6 Gew.%, des sauren Veresterungskatalysators zur Anwendung. Bei der Umsetzung des Isosorbids mit

der aliphatischen Carbonsäure kann das Molverhältnis zwischen Isosorbid und der aliphatischen Carbonsäure in weiten Grenzen schwanken. Insbesondere wenn die Umsetzung in einem geeigneten Lösungsmittel durchgeführt wird, beträgt das Molverhältnis Isosorbid : aliphatischer Carbonsäure normalerweise 1 : (0,7 bis 1,3), vorzugsweise 1 : (0,8 bis 1,2). Bei der Umsetzung kann jedoch sowohl der Isosorbid als auch die aliphatische Carbonsäure in einem großen molaren, d.h. z.B. 2- bis 20fachen und noch größeren Überschuß anwesend sein. Bei Anwendung eines großen Überschusses von Isosorbid wird nach der Umsetzung mit der aliphatischen Carbonsäure das erhaltene Gemisch in nichtumgesetztes Isosorbid und Acylierungsprodukt getrennt. Dies erfolgt z.B. dadurch, daß man den überschüssigen Isosorbid durch Extraktion mit Wasser aus dem Reaktiongsgemisch entfernt oder indem man die Acylierungsprodukte durch Extraktion mit einem geeigneten Lösungsmittel, wie Methylenchlorid oder Essigsäurethylester, aus dem Reaktionsgemisch entfernt.

Bei der Anwendung eines großen molaren Überschusses der aliphatischen Carbonsäure muß verhindert werden, daß sich größere Mengen an Isosorbid-diacylat bilden. Dies wird in einfacher Weise erreicht durch Zusatz eines zur azeotropen Abdestillation des entstehenden Reaktionswassers geeigneten Lösungsmittels in ausreichender Menge und Abbruch der Umsetzung nach Bildung der theoretischen Menge Wasser.

Die Umsetzung zwischen Isosorbid und der aliphatischen Carbonsäure wird bei Raumtemperatur oder zweckmäßigerweise bei erhöhter Temperatur, insbesondere bei der Rückflußtemperatur des verwendeten Lösungsmittels durchgeführt. Dabei findet eine Acylierung oder Veresterung des Isosorbids unter Abspaltung von Wasser statt. Das Fortschreiten der Reaktion kann deshalb durch Bestimmung des abgeschiedenen Wassers verfolgt werden. Bei der erfindungsgemäßen, sauer katalysierten Veresterung von Isosorbid mit aliphatischen Carbonsäuren, insbesondere Alkancarbonsäuren mit 1 bis 7 C-Atomen, vorzugsweise 2 bis 4 C-Atomen, wird überraschenderweise praktisch kein Isosorbid-5-acylat, sondern als Hauptprodukt Isosorbid-2-acylat gebildet, das als einziges Nebenprodukt Isosorbid-2,5-diacylat in untergeordnetem Maße enthält. Das in untergeordnetem Maße gebildete Isosorbid-2,5-diacylat stört den Verlauf der weiteren Umsetzung nicht, kann jedoch gewünschtenfalls auch durch Destillation oder Kristallisation abgetrennt werden. Die Tatsache, daß bei der sauer katalysierten Umsetzung des Isosorbids mit aliphatischen Carbonsäuren als Monoacylat praktisch nur das Isosorbid-2-acylat gebildet wird, war nicht vorhersehbar, denn in dem bei der sauer katalysierten Umsetzung von Isosorbid mit Alkancarbonsäureanhydriden oder -chloriden oder -bromiden entstehenden Acylierungsgemisch ist, bezogen auf Isosorbid-2-acylat, mindestens die doppelte Menge Isosorbid-5-acylat vorhanden (vgl. DE-OS 27 51 934).

Das bei der sauer katalysierten Umsetzung von Isosorbid mit einer aliphatischen Carbonsäure erhaltene Acylierungsgemisch, das neben Isosorbid-2-acylat als Nebenprodukt Isosorbid-2,5-diacylat und höchstens Spuren von Isosorbid-5-acylat enthält, wird nun direkt oder vorzugsweise nach Abtrennung des Isosorbid-2,5-diacylats in an sich bekannter Weise nitriert. Diese Nitrierung wird zweckmäßigerweise in einem geeigneten Lösungsmittel, wie z.B. Methylenchlorid, bei Temperaturen von -10 bis 30°C, vorzugsweise von 0 bis 20°C, mit einem aus Acetanhydrid/Eisessig/Salpetersäure oder Schwefelsäure/Salpetersäure oder Essigsäure/Salpetersäure bestehenden Nitriergemisch durchgeführt.

Das bei der Nitrierung erhaltene Gemisch, bestehend aus Isosorbid-2-acylat-5-nitrat und Isosorbid-2,5-diacylat wird nun ohne Zwischenisolierung in an sich bekannter Weise hydrolysiert und/oder umgeestert, wodurch durch Abspaltung der Acylgruppen als Hauptprodukt Isosorbid-5-nitrat und etwas Isosorbid entsteht. Der Isosorbid kann problemlos durch Extraktion mit Wasser entfernt werden.

Die Hydrolyse wird am einfachsten mit Hilfe einer wäßrigen Alkalihydroxydlösung, z.B. einer wäßrigen Natriumhydroxydlösung, bei Temperaturen von 0 bis 50°C, vorzugsweise 0 bis 40°C, durchgeführt. Die Umesterung wird bei Temperaturen von 10 bis 60°C, vorzugsweise 20 bis 60°C in einem Alkohol, insbesondere einem Alkohol mit 1 bis 4 C-Atomen, vorzugsweise in Methanol oder Ethanol, in Gegenwart katalytischer Mengen einer Base, beispielsweise eines Alkalihydroxyds, wie Natrium- oder Kaliumhydroxyd, oder eines Alkalialkoholats, wie z.B. Natriummethylat, durchgeführt. Bei Verwendung wäßriger Alkohole verlaufen Umesterung und Hydrolyse nebeneinander ab.

Aus dem bei der sauer katalysierten Umsetzung des Isosorbids mit der aliphatischen Carbonsäure entstehenden Acylierungsprodukt, das Isosorbid-2-acylat, als Nebenprodukt Isosorbid-2,5-diacylat und nur Spuren von Isosorbid-5-acylat enthält, wird das Diacylat vor der Weiterverarbeitung vorzugsweise abgetrennt. Dies kann durch eine Rektifikation bei vermindertem Druck, z.B. bei einem Druck von 0,05 bis 1 mbar, vorzugsweise von 0,1 bis 0,3 mbar, erfolgen, wobei als erste Fraktion das Isosorbid-2-acylat übergeht. Das Isosorbid-2-acylat kann aus dem Acylierungsgemisch aber auch auf andere Weise, z.B. durch Kristallisation, abgetrennt werden. Das abgetrennte Isosorbid-2-acylat wird dann, wie bereits beschrieben, nitriert und anschließend hydrolysiert und/oder umgeestert.

Das erfindungsgemäße Verfahren liefert reines Isosorbid-5-nitrat in einer Ausbeute bis zu 50 %.

Die folgenden Beispiele erläutern das erfindungsgemäße Verfahren.

Beispiel 1

146 g Isosorbid, 60 g Eisessig und 5g p-Toluolsulfonsäure werden in 700ml Ethylenchlorid am Rückfluß unter Einschaltung eines Wasserabscheiders gekocht, bis sich kein Wasser mehr abscheidet. Dann wird die Reaktionsmischung eingeengt, mit 400 ml Methylenchlorid aufgenommen und zweimal mit je 100 ml wäßriger konzentrierter Natriumhydrogencarbonatlösung ausgeschüttelt. Die wäßrigen Phasen werden mit zweimal je 200 ml Methylenchlorid ausgeschüttelt. Die Methylenchloridphasen werden vereinigt und mit Natriumsulfat getrocknet. Die Methylenchloridlösung wird nun auf ca. 10°C abgekühlt. Die auf -5°C vorgekühlte Nitriermischung - hergestellt aus auf -5°C abgekühltem Acetanhydrid (108 g) und Eisessig(64 g) durch Zutropfen von 61 g 100%iger Salpetersäure bei -5 bis 0°C - wird nun unter Umrühren zugetropft, wobei die Innentemperatur unter 20°C gehalten wird. Nach 2 Stunden langem Nachrühren bei 20°C wird die Mischung auf ca. 1,2 l Eiswasser gegossen, die organische Phase abgetrennt, die wäßrige Phase einmal mit 200 ml Methylenchlorid, und die vereinigten organischen Phasen mit 300 ml Wasser ausgeschüttelt und eingeengt (dünnschichtchromatographisch kann nachgewiesen werden, daß kein explosives Isosorbiddinitrat zugegen ist). Der Rückstand wird in 700 ml Ethanol gelöst und auf 50°C erwärmt, mit einer Lösung von 10 g Kaliumhydroxyd in 100 ml Ethanol versetzt und das Gemisch 10 Minuten bei 50°C gerührt. Nach Abkühlen auf 20°C wird mit verdünnter Salzsäure neutralisiert und eingeengt. Der Rückstand wird mit 500 ml Wasser aufgenommen und viermal mit je 100 ml Methylenchlorid ausgeschüttelt. Die Methylenchloridphasen werden vereinigt, über Natriumsulfat getrocknet und eingeengt. Der Rückstand wird aus einer Mischung von 3 Teilen Chloroform und 1 Teil Tetrachlorkohlenstoff umkristallisiert und ergibt 80,2 g Isosorbid-5-nitrat in Form farbloser filziger Nadeln vom Fp. 90 bis 91°C.
Ausbeute : 42,0 % der Theorie.

Beispiel 2

146 g Isosorbid, 80 g Isobuttersäure und 5 ml konzentrierte Schwefelsäure werden in einer Mischung aus 400 ml Chlorbenzol und 200 ml Toluol am Rückfluß unter Einschaltung eines Wasserabscheiders erhitzt, bis sich kein Wasser mehr abscheidet.

Nach dem Abkühlen auf 20°C wird zweimal mit je 300 ml Wasser ausgeschüttelt und die vereinigten wäßrigen Phasen mit 200 ml Methylenchlorid ausgeschüttelt, die organischen Phasen werden vereinigt, über Natriumsulfat getrocknet und eingeengt. Die Destillation dieses Rückstands bei 0,4 mbar ergibt 115 g einer bei 117 bis 120°C siedenden Fraktion, die aus Isosorbid-2-isobutylat besteht. Diese Fraktion wird, wie im Beispiel 1 beschrieben, weiter umgesetzt und liefert 66,7 g Isosorbid-5-nitrat vom Fp. 90 bis 91°C.
Ausbeute: 34,9 %.

<u>Beispiel 3</u>

Die, wie in Beispiel 2 beschrieben, bei 117 bis 120°C /0,4 mbar siedende Fraktion wird zu einer auf 10 bis 15°C vorgekühlten Nitriermischung (2,5 g Harnstoff, 64 ml konzentrierter Salpetersäure (D = 1,42), 75 ml konzentrierter Schwefelsäure) getropft. Dann wird die Mischung 20 Minuten lang bei 15 bis 20°C gerührt, auf 30 ml Eiswasser gegossen und das sich abscheidende Produkt in 400 ml Methylenchlorid aufgenommen. Nach Zugabe von 500 ml Toluol wird die Lösung auf das halbe Volumen eingeengt. Diese Toluollösung wird mit 500 ml 1n wäßriger Natronlauge versetzt und 18 Stunden lang bei 20°C gerührt. Die wäßrige Phase wird abgetrennt, die Toluolphase mit 300 ml Petroläther verdünnt und einmal mit Wasser ausgeschüttelt und verworfen. Aus den vereinigten wäßrigen Phasen wird das Isosorbid-5-nitrat durch Ausschütteln mit Methylenchlorid und Umkristallisieren aus Toluol gewonnen. Schmelzpunkt: 90 bis 91°C.
Ausbeute: 51,3 g (26,9 %).

<u>Beispiel 4</u>

146 g Isosorbid, 60 g Eisessig und 5 g Kaliumhydrogensulfat werden in einer Mischung aus 400 ml Diethylenglykoldimethylether und 200 ml Toluol am Rückfluß unter Einschaltung eines Wasserabscheiders erhitzt, bis sich kein Wasser mehr abscheidet. Die Reaktionsmischung wird im Wasserstrahlvakuum eingeengt und der Rückstand mit 400 ml Wasser aufgenommen und einmal mit 150 ml Diethylether ausgeschüttelt. Aus der

wäßrigen Phase wird darauf durch viermaliges Ausschütteln mit Methylenchlorid das Isosorbid-2-acetat ausgeschüttelt. Nach dem Einengen wird aus wenig Isopropanol umkristallisiert. Ausbeute: 109 g farblose Nadeln, Fp.= 77 bis 78°C (57,9 % der Theorie). Dieses Produkt wird in der im Beispiel 1 angegebenen Weise nitriert und entacetyliert und liefert 91,5 g Isosorbid-5-nitrat vom Fp. = 90 bis 91°C. Gesamtausbeute: 48,0 %.

PATENTANSPRÜCHE

1. Verfahren zur Herstellung von 1,4-3,6-Dianhydro-D-sorbit-5-nitrat der Formel I

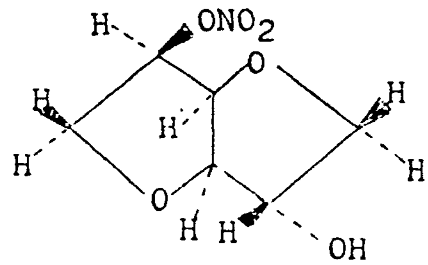

(I)

auch Isosorbid-5-nitrat genannt, dadurch gekennzeichnet, daß 1,4-3,6-Dianhydro-D-sorbit, auch Isosorbid genannt, mit einer aliphatischen Carbonsäure unter Bildung eines Acylierungsgemisches umgesetzt wird, das als Hauptprodukt Isosorbid-2-acylat, als Nebenprodukt Isosorbid-2,5-diacylat und nur Spuren Isosorbid-5-acylat enthält, und daß das Acylierungsgemisch anschließend nitriert und das erhaltene Nitrierungsgemisch zur Abspaltung der Acylgruppen hydrolysiert und/oder umgeestert wird.

2. Verfahren nach Anspruch 1, dadurch gekennzeichnet, daß der Isosorbid mit einer aliphatischen Carbonsäure mit 1 bis 7 C-Atomen umgesetzt wird.

3. Verfahren nach den Ansprüchen 1 oder 2, dadurch gekennzeichnet, daß der Isosorbid mit einer Alkancarbonsäure mit 2 bis 4 C-Atomen umgesetzt wird.

4. Verfahren nach den Ansprüchen 1 bis 3, dadurch gekennzeichnet, daß der Isosorbid mit der aliphatischen Carbonsäure in Gegenwart eines sauren Katalysators umgesetzt wird.

5. Verfahren nach den Ansprüchen 1 bis 4, dadurch gekennzeichnet, daß der Isosorbid mit der aliphatischen Carbonsäure in Gegenwart einer Sulfonsäure, einer Mineralsäure oder

eines Alkalihydrogensulfats als sauren Katalysators umgesetzt wird.

6. Verfahren nach den Ansprüchen 1 bis 5, dadurch gekennzeichnet, daß die Umsetzung des Isosorbids mit der aliphatischen Carbonsäure in einem Lösungsmittel durchgeführt wird, insbesondere einem solchen, das zur azeotropen Abdestillation des bei der Umsetzung entstehenden Reaktionswassers geeignet ist.

7. Verfahren nach den Ansprüchen 1 bis 6, dadurch gekennzeichnet, daß aus dem bei der Umsetzung des Isosorbids mit der aliphatischen Carbonsäure erhaltenen Acylierungsgemisch das Isosorbid-2-acylat abgetrennt und der anschließenden Nitrierung und darauf folgender Hydrolyse und/oder Umesterung unterworfen wird.

8. Verfahren nach den Ansprüchen 1 bis 7, dadurch gekennzeichnet, daß die Nitrierung in einem Lösungsmittel bei Temperaturen von 0 bis 20°C durchgeführt wird.

9. Verfahren nach den Ansprüchen 1 bis 7, dadurch gekennzeichnet, daß das erhaltene Nitrierungsgemisch zur Abspaltung der Acylgruppen mit einer wäßrigen Alkalihydroxydlösung bei Temperaturen von 0 bis 50°C, vorzugsweise 0 bis 40°C, hydrolysiert wird.

10. Verfahren nach den Ansprüchen 1 bis 9, dadurch gekennzeichnet, daß das erhaltene Nitriergemisch zur Abspaltung der Acylgruppen mit einem Alkohol bei Temperaturen von 10 bis 60°C, vorzugsweise 20 bis 60°C, in Gegenwart einer katalytischen Menge einer Base umgeestert wird.